# EUROPÄISCHE PATENTSCHRIFT

(11) **EP 2 311 392 B1**
(45) Veröffentlichungstag und Bekanntmachung des Hinweises auf die Patenterteilung: **06.07.2016**
(21) Anmeldenummer: 10187643.1
(22) Anmeldetag: 14.10.2010
(51) Int. Cl.: A61B 17/29, A61B 18/14, A61B 17/00

(54) **Chirurgisches Instrument**
Surgical Instrument
Instrument chirurgical

(30) Priorität: 15.10.2009 DE 102009045749
(43) Veröffentlichungstag der Anmeldung: 20.04.2011
(73) Patentinhaber: AESCULAP AG, 78532 Tuttlingen (DE)
(72) Erfinder: Schweitzer, Tom, 78532, Tuttlingen (DE); Mayenberger, Rupert, 78239, Rielasingen (DE)
(74) Vertreter: Hoeger, Stellrecht & Partner Patentanwälte mbB

(56) Entgegenhaltungen:
- WO-A1-2009/064807
- WO-A2-03/030708
- DE-U1-202007 007 327
- US-A1- 2008 004 656
- US-B1- 6 299 625

## Beschreibung

Die vorliegende Erfindung betrifft ein chirurgisches Instrument, welches mindestens zwei relativ zueinander bewegbare Werkzeugelemente, die an einem distalen Ende einer Führungseinrichtung des chirurgischen Instruments angeordnet sind, eine Kraftübertragungseinrichtung zum Übertragen einer Betätigungskraft von einem proximalen Ende der Kraftübertragungseinrichtung zu einem distalen Ende der Kraftübertragungseinrichtung zum Bewegen mindestens eines der mindestens zwei Werkzeugelemente relativ zu der Führungseinrichtung und eine Kopplungseinrichtung zum wahlweisen direkten oder indirekten Koppeln der Kraftübertragungseinrichtung mit mindestens einem der mindestens zwei Werkzeugelemente umfasst.

Derartige chirurgische Instrumente sind aus dem Stand der Technik bekannt. Beispielsweise werden in der US 2008/0004656 A1 und in der WO 2009/064807 A1 chirurgische Instrumente mit zwei relativ zueinander bewegbaren Werkzeugelementen beschrieben. Insbesondere werden hierbei beispielsweise zwei Kraftübertragungseinrichtungen zum Übertragen einer Betätigungskraft auf zwei Werkzeugelemente verwendet, wobei jedem Werkzeugelement eine Kraftübertragungseinrichtung zugeordnet ist. Insbesondere bei beispielsweise als Rohrschaftinstrumenten ausgebildeten chirurgischen Instrumenten werden die beiden Kraftübertragungseinrichtungen zum Übertragen einer Betätigungskraft auf die Werkzeugelemente dabei beispielsweise nebeneinander liegend in dem Rohrschaft geführt und müssen auf Grund des vorzugsweise geringen Durchmessers des Rohrschafts besonders dünn ausgebildet sein. Wird nun mittels der Kraftübertragungseinrichtung eine Betätigungskraft auf das Werkzeugelement ausgeübt, so kann es bei den aus dem Stand der Technik bekannten chirurgischen Instrumenten vorkommen, dass sich eine Kraftübertragungseinrichtung verformt und somit eine gezielte Kraftübertragung auf das Werkzeugelement erschwert.

Der vorliegenden Aufgabe liegt die Erfindung zu Grunde, ein chirurgisches Instrument bereit zu stellen, welches eine besonders einfache Handhabung und eine direkte Kraftübertragung auf mindestens zwei relativ zueinander bewegbare Werkzeugelemente ermöglicht.

Diese Aufgabe wird erfindungsgemäß dadurch gelöst, dass die Kraftübertragungseinrichtung aus einer ersten Kopplungsstellung, in welcher die Kraftübertragungseinrichtung mit einem ersten Werkzeugelement des chirurgischen Instruments gekoppelt ist, in eine zweite Kopplungsstellung, in welcher die Kraftübertragungseinrichtung mit einem zweiten Werkzeugelement des chirurgischen Instruments gekoppelt ist, bringbar ist.

Dadurch, dass das chirurgische Instrument eine Kopplungseinrichtung zum wahlweisen Koppeln der Kraftübertragungseinrichtung mit mindestens einem der mindestens zwei Werkzeugelemente umfasst, kann bei dem chirurgischen Instrument eine einzige Kraftübertragungseinrichtung vorgesehen sein. Es ist somit nicht notwendig, für jedes der Werkzeugelemente des chirurgischen Instruments eine separate Kraftübertragungseinrichtung vorzusehen.

Ferner kann dadurch, dass das chirurgische Instrument eine Kopplungseinrichtung zum wahlweisen direkten oder indirekten Koppeln der Kraftübertragungseinrichtung mit einem der mindestens zwei Werkzeugelemente umfasst, die Kraftübertragungseinrichtung stabil ausgebildet sein, da ein zusätzlicher Platz für eine zusätzliche Kraftübertragungseinrichtung an dem chirurgischen Instrument nicht erforderlich ist. Auf diese Weise kann mittels der Kraftübertragungseinrichtung eine Betätigungskraft von einem proximalen Ende der Kraftübertragungseinrichtung zu einem distalen Ende der Kraftübertragungseinrichtung zum Bewegen mindestens eines der mindestens zwei Werkzeugelemente relativ zu der Führungseinrichtung übertragen werden, wobei auch bei einer größeren Betätigungskraft keine oder nur eine geringe Verformung der Kraftübertragungseinrichtung erfolgt. Auf diese Weise ist eine besonders einfache Handhabung des chirurgischen Instruments und eine besonders direkte Kraftübertragung auf die mindestens zwei Werkzeugelemente des chirurgischen Instruments möglich.

Günstig ist es, dass die Kraftübertragungseinrichtung aus einer ersten Kopplungsstellung, in welcher die Kraftübertragungseinrichtung mit einem ersten Werkzeugelement des chirurgischen Instruments gekoppelt ist, in eine zweite Kopplungsstellung, in welcher die Kraftübertragungseinrichtung mit einem zweiten Werkzeugelement des chirurgischen Instruments gekoppelt ist, bringbar ist. Auf diese Weise kann besonders einfach zwischen der ersten Kopplungsstellung zum Betätigen des ersten Werkzeugelements und der zweiten Kopplungsstellung zum Betätigen des zweiten Werkzeugelements umgeschaltet werden. Die Kraftübertragungseinrichtung ist hierbei vorzugsweise ausschließlich mit jeweils einem der beiden Werkzeugelemente gekoppelt, wobei das andere Werkzeugelement günstigerweise in einer Ruhestellung festgelegt ist.

Bei einer Ausgestaltung der Erfindung ist vorgesehen, dass die Kraftübertragungseinrichtung zumindest teilweise innerhalb der Führungseinrichtung angeordnet ist.

Alternativ hierzu kann vorgesehen sein, dass die Führungseinrichtung zumindest teilweise innerhalb der Kraftübertragungseinrichtung angeordnet ist.

Die Kraftübertragungseinrichtung ist vorzugsweise drehbar an der Führungseinrichtung gelagert. Eine solche Lagerung ist insbesondere dann vorteilhaft, wenn die Kopplungseinrichtung des chirurgischen Instruments durch eine Drehung der Kraftübertragungseinrichtung relativ zu der Führungseinrichtung betätigbar ist.

Ferner ist die Kraftübertragungseinrichtung vorzugsweise zumindest teilweise elektrisch leitend ausgebildet. Auf diese Weise kann die Kraftübertragungseinrichtung als Stromübertragungseinrichtung zum Übertragen eines elektrischen Stroms auf mindestens eines der mindestens zwei Werkzeugelemente des chirurgischen Instruments verwendet werden.

Bei einer bevorzugten Ausgestaltung der Erfindung kann vorgesehen sein, dass die Kraftübertragungseinrichtung in mindestens einer Richtung senkrecht zu einer Kraftübertragungsrichtung flexibel ausgebildet ist. Insbesondere dann, wenn die Führungseinrichtung nicht als geradlinige Führungseinrichtung zum geradlinigen Führen der Kraftübertragungseinrichtung ausgebildet ist, kann auf diese Weise eine Anpassung der Form der Kraftübertragungseinrichtung an die Form der Führungseinrichtung erfolgen.

Vorzugsweise füllt die Kraftübertragungseinrichtung einen in der Führungseinrichtung gebildeten Hohlraum zumindest teilweise, insbesondere im Wesentlichen vollständig, aus. Auf diese Weise ist eine besonders sichere Führung der Kraftübertragungseinrichtung an der Führungseinrichtung gewährleistet. Ferner ist dadurch eine direkte Kraftübertragung auf die mindestens zwei Werkzeugelemente des chirurgischen Instruments möglich, da die Kraftübertragungseinrichtung sich innerhalb der Führungseinrichtung nicht verformen kann.

Günstig ist es, wenn das chirurgische Instrument einen Handgriff umfasst, an welchem ein Chirurg zur Betätigung des chirurgischen Instruments besonders einfach angreifen kann. Der Handgriff ist vorzugsweise drehfest an der Führungseinrichtung angeordnet. Alternativ oder ergänzend hierzu kann jedoch auch vorgesehen sein, dass der Handgriff drehfest an der Kraftübertragungseinrichtung angeordnet ist. Wesentlich hierbei ist, dass entweder die Führungseinrichtung oder die Kraftübertragungseinrichtung relativ zu dem Handgriff drehfest angeordnet ist. Durch eine Drehung des jeweils anderen Elements relativ zu dem Handgriff kann dann eine besonders einfache Betätigung der Kopplungseinrichtung erfolgen.

Besonders günstig ist es, wenn das chirurgische Instrument eine Betätigungseinrichtung zum Wechseln einer Kopplungsstellung der Kopplungseinrichtung, insbesondere zum Drehen der Führungseinrichtung relativ zu dem Handgriff, umfasst. Auf diese Weise ist eine besonders einfache Betätigung der Kopplungseinrichtung und ferner ein besonders einfacher Aufbau des Handgriffs des chirurgischen Instruments möglich. Die Betätigungseinrichtung ist vorzugsweise an einem dem Handgriff zugewandten, proximalen Ende der Führungseinrichtung angeordnet.

Günstig ist es, wenn die Führungseinrichtung starr ausgebildet ist. Auf diese Weise ist eine besonders stabile Führung der Kraftübertragungseinrichtung an der Führungseinrichtung möglich.

Ferner ist die Führungseinrichtung vorzugsweise zumindest näherungsweise hohlzylindrisch, insbesondere als ein, vorzugsweise langgestreckter, Schaft ausgebildet. Auf diese Weise können mittels des chirurgischen Instruments auch schwer zugängliche Stellen im Körper erreicht werden.

Vorteilhaft kann es sein, wenn die Führungseinrichtung zumindest teilweise elektrisch leitend ausgebildet ist. Auf diese Weise kann mittels der Führungseinrichtung besonders einfach ein elektrischer Strom auf mindestens ein Werkzeugelement des chirurgischen Instruments übertragen werden. Insbesondere kann vorgesehen sein, dass die Führungseinrichtung als eine erste Stromleitung und die Kraftübertragungseinrichtung als eine zweite Stromleitung zur Versorgung der mindestens zwei Werkzeugelemente des chirurgischen Instruments mit Strom ausgebildet sind.

Die Führungseinrichtung und die Kraftübertragungseinrichtung sind vorzugsweise elektrisch voneinander isoliert.

Die Kraftübertragungseinrichtung ist vorzugsweise wahlweise ausschließlich mit einem ersten Werkzeugelement oder mit einem zweiten Werkzeugelement koppelbar. Auf diese Weise kann verhindert werden, dass durch ein Aufbringen einer Betätigungskraft auf die Kraftübertragungseinrichtung die mindestens zwei relativ zueinander bewegbaren Werkzeugelemente des chirurgischen Instruments gleichzeitig betätigt werden.

Günstig ist es, wenn die Kopplungseinrichtung im Wesentlichen drehfest an der Führungseinrichtung angeordnet ist. Auf diese Weise kann eine Änderung der Kopplungsstellung der Kraftübertragungseinrichtung besonders einfach durch eine Drehung der Kraftübertragungseinrichtung relativ zu der Führungseinrichtung beziehungsweise durch eine Drehung der Führungseinrichtung relativ zu der Kraftübertragungseinrichtung erfolgen.

Bei einer Ausgestaltung der Erfindung kann vorgesehen sein, dass das chirurgische Instrument eine elektrische Umschalteinrichtung zum wahlweisen Beaufschlagen mindestens eines der mindestens zwei Werkzeugelemente mit elektrischer Energie umfasst. Mindestens eines der mindestens zwei Werkzeugelemente ist hierzu beispielsweise mittels der Umschalteinrichtung mit einem Anschluss zum Anlegen einer elektrischen Spannung direkt oder indirekt elektrisch leitend in Eingriff bringbar. Insbesondere kann hierbei vorgesehen sein, dass ausschließlich jeweils nur eines der mindestens zwei relativ zueinander bewegbaren Werkzeugelemente des chirurgischen Instruments mit elektrischer Energie beaufschlagt wird.

Zur Übertragung von elektrischer Energie auf das mindestens eine der mindestens zwei Werkzeugelemente umfasst das mindestens eine der mindestens zwei Werkzeugelemente mindestens eine Kontaktstelle zum direkten Übertragen von elektrischer Energie und/oder mindestens eine Kopplungsstelle zum kontaktfreien Übertragen einer Wechselspannung auf das mindestens eine der mindestens zwei Werkzeugelemente.

Die elektrische Umschalteinrichtung ist vorzugsweise an dem distalen Ende der Führungseinrichtung angeordnet. Dadurch kann ein elektrischer Strom von dem proximalen Ende der Führungseinrichtung zu dem distalen Ende der Führungseinrichtung mit einer einzigen elektrischen Leitung geführt werden. Eine Kopplung dieser einen elektrischen Leitung mit dem jeweils mit elektrischer Energie zu beaufschlagenden mindestens einen der mindestens zwei Werkzeugelemente erfolgt dann erst am distalen Ende der Führungseinrichtung.

Bei einer Ausgestaltung der Erfindung ist vorgesehen, dass die Kopplungseinrichtung zumindest abschnittsweise elektrisch leitend ausgebildet ist zum Übertragen eines elektrischen Stroms von der Kraftübertragungseinrichtung auf mindestens eines der mindestens zwei Werkzeugelemente. Die Kopplungseinrichtung umfasst dann vorzugsweise die elektrische Umschalteinrichtung oder bildet die elektrische Umschalteinrichtung, so dass insbesondere bei einer Umschaltung der Kopplungsstellung zur mechanischen Kopplung des mindestens einen Werkzeugelements mit der Kraftübertragungseinrichtung gleichzeitig eine elektrische Verbindung zwischen der Kraftübertragungseinrichtung und dem gekoppelten Werkzeug mittels der Kopplungseinrichtung hergestellt wird.

Die Kopplungseinrichtung ist vorzugsweise ausgebildet zum Übertragen eines elektrischen Stroms von der Kraftübertragungseinrichtung auf das erste Werkzeugelement in einer ersten Kopplungsstellung der Kraftübertragungseinrichtung, in welcher die Kraftübertragungseinrichtung mit einem ersten Werkzeugelement gekoppelt ist, und von der Kraftübertragungseinrichtung auf das zweite Werkzeugelement in einer zweiter Kopplungsstellung, in welcher die Kraftübertragungseinrichtung mit einem zweiten Werkzeugelement gekoppelt ist. Da die Kopplungseinrichtung somit sowohl eine mechanische Kopplung als auch eine elektrische Kopplung der Kraftübertragungseinrichtung mit mindestens einem der mindestens zwei Werkzeugelemente ermöglicht, ist eine separate elektrische Umschalteinrichtung bei dieser Ausführungsform entbehrlich.

Bei einer Ausgestaltung der Erfindung ist vorgesehen, dass die Kopplungseinrichtung mindestens ein Kopplungsglied umfasst, welches mit mindestens einem Kopplungsglied der Kraftübertragungseinrichtung in Eingriff bringbar ist zum Herstellen einer formschlüssigen Verbindung zwischen der Kopplungseinrichtung und der Kraftübertragungseinrichtung in einer Kraftübertragungsrichtung der Kraftübertragungseinrichtung.

Auf diese Weise ist eine besonders stabile Verbindung zwischen der Kraftübertragungseinrichtung und der Kopplungseinrichtung in der Kraftübertragungsrichtung möglich, so dass mittels der Kraftübertragungseinrichtung und der Kopplungseinrichtung eine Betätigungskraft zuverlässig auf mindestens eines der mindestens zwei Werkzeugelemente des chirurgischen Instruments übertragbar ist.

Das mindestens eine Kopplungsglied der Kopplungseinrichtung und das mindestens eine Kopplungsglied der Kraftübertragungseinrichtung sind dabei vorzugsweise so ausgebildet und so relativ zueinander angeordnet, dass sie durch eine Drehung des mindestens einen Kopplungsglied der Kopplungseinrichtung relativ zu dem mindestens einen Kopplungsglied der Kraftübertragungseinrichtung miteinander in Eingriff und außer Eingriff bringbar sind. Um eine besonders störungsfreie und zuverlässige Handhabung des chirurgischen Instruments zu gewährleisten, kann vorgesehen sein, dass die Kopplungseinrichtung mindestens eine beispielsweise als Einführschräge ausgebildete Kopplungshilfe zum Vereinfachen der Kopplung der Kraftübertragungseinrichtung mit mindestens einem der mindestens zwei Werkzeugelemente aufweist. Beispielsweise kann hierzu vorgesehen sein, dass mindestens ein Kopplungsglied der Kopplungseinrichtung und/oder mindestens ein Kopplungsglied der Kraftübertragungseinrichtung eine Einführschräge aufweist, so dass sich das mindestens eine Kopplungsglied der Kopplungseinrichtung und das mindestens eine Kopplungsglied der Kraftübertragungseinrichtung beispielsweise bei einer Drehbewegung des mindestens einen Kopplungsglieds der Kopplungseinrichtung relativ zu dem mindestens einen Kopplungsglied der Kraftübertragungseinrichtung nicht verhakt und dadurch eine Umschaltung der Kopplungsstellung erschwert.

Bei einer Ausgestaltung der Erfindung kann vorgesehen sein, dass die Kraftübertragungseinrichtung direkt mit mindestens einem der mindestens zwei Werkzeugelemente koppelbar ist. Hierzu kann vorgesehen sein, dass die mindestens zwei Werkzeugelemente des chirurgischen Instruments jeweils mindestens ein Kopplungsglied der Kopplungseinrichtung umfassen, vorzugsweise jeweils einstückig mit mindestens einem Kopplungsglied der Kopplungseinrichtung ausgebildet sind.

Bei einer weiteren Ausgestaltung der Erfindung kann vorgesehen sein, dass die Kopplungseinrichtung mindestens ein Kopplungselement zum Koppeln der Kraftübertragungseinrichtung mit mindestens einem der mindestens zwei Werkzeugelemente umfasst. Auf diese Weise ist eine indirekte Kopplung der Kraftübertragungseinrichtung mit mindestens einem der mindestens zwei Werkzeugelemente des chirurgischen Instruments möglich.

Das mindestens eine Kopplungselement greift hierzu vorzugsweise an einem distalen Ende der Kraftübertragungseinrichtung an.

Bei einer Ausgestaltung der Erfindung kann vorgesehen sein, dass die Kopplungseinrichtung für jedes relativ zu der Führungseinrichtung bewegbare Werkzeugelement des chirurgischen Instruments mindestens ein Kopplungselement umfasst.

Günstig ist es, wenn die Kopplungseinrichtung mindestens zwei elektrisch voneinander isolierte Kopplungselemente umfasst. Auf diese Weise können den mindestens zwei Werkzeugelementen des chirurgischen Instruments, welche beispielsweise mittels jeweils eines Kopplungselements mit der Kraftübertragungseinrichtung koppelbar sind, unabhängig voneinander mit Strom beaufschlagt werden.

Mindestens ein Kopplungselement der Kopplungseinrichtung ist vorzugsweise zumindest teilweise innerhalb der Führungseinrichtung angeordnet. Insbesondere dann, wenn ein Kopplungsglied der Kraftübertragungseinrichtung und ein Kopplungsglied des mindestens einen Kopplungselements vollständig innerhalb der Führungseinrichtung angeordnet sind, ist eine Verunreinigung der Kopplungsglieder und eine dadurch verursachte erschwerte Betätigung der Kopplungseinrichtung wirksam verhindert.

Bei einer Ausgestaltung der Erfindung kann vorgesehen sein, dass mindestens zwei Kopplungselemente symmetrisch um eine Mittelachse der Führungseinrichtung verteilt angeordnet sind.

Ferner kann vorgesehen sein, dass mindestens zwei Kopplungselemente auf einander gegenüberliegenden Seiten einer Längsmittelebene der Führungseinrichtung angeordnet sind.

Mindestens ein Kopplungselement der Kopplungseinrichtung ist vorzugsweise in einer Kraftübertragungsrichtung der Kraftübertragungseinrichtung verschieblich an der Führungseinrichtung gelagert. Auf diese Weise kann eine mittels der Kraftübertragungseinrichtung auf das mindestens eine Kopplungselement übertragene, in der Kraftübertragungsrichtung wirkende Betätigungskraft besonders einfach mittels des mindestens einen Kopplungselements auf mindestens eines der mindestens zwei Werkzeugelemente des chirurgischen Instruments übertragen werden. Durch eine geeignete Kopplung des mindestens einen Kopplungselements mit dem jeweiligen mindestens einen der mindestens zwei Werkzeugelemente wird die Bewegung des mindestens einen Kopplungselements in der Kraftübertragungsrichtung dann vorzugsweise in eine Bewegung des mindestens einen der mindestens zwei Werkzeugelemente in der Kraftübertragungsrichtung oder in eine Drehbewegung des mindestens einen der mindestens zwei Werkzeugelemente um eine quer, vorzugsweise im Wesentlichen senkrecht zu der Kraftübertragungsrichtung ausgerichtete Drehachse des mindestens einen der mindestens zwei Werkzeugelemente übersetzt.

Bei einer Ausgestaltung der Erfindung kann vorgesehen sein, dass die Kopplungseinrichtung mindestens eine Führungsbahn zum Führen mindestens eines bewegbaren Werkzeugelements umfasst. Dadurch kann insbesondere ein Führungselement des mindestens einen bewegbaren Werkzeugelements auf einer vorgegebenen Bahn bewegt werden. Durch eine geeignete Form der Führungsbahn und/oder des Führungselements kann das mindestens eine bewegbare Werkzeugelement beispielsweise um eine Drehachse gedreht werden.

Günstig ist es, wenn das chirurgische Instrument mindestens ein bezüglich der Führungseinrichtung feststehendes Werkzeugelement umfasst.

Hierbei bildet vorzugsweise mindestens eines der mindestens zwei relativ zu der Führungseinrichtung bewegbaren Werkzeugelemente zusammen mit dem mindestens einen bezüglich der Führungseinrichtung feststehenden Werkzeugelement vorzugsweise ein Werkzeug des chirurgischen Instruments, beispielsweise ein Klemmwerkzeug und/oder ein Schneidwerkzeug. Eine Betätigung des Werkzeugs, beispielsweise ein Durchführen eines Klemmvorgangs und/oder eines Schneidvorgangs, erfolgt hierbei vorzugsweise dadurch, dass das mindestens eine relativ zu der Führungseinrichtung bewegbare Werkzeugelement relativ zu der Führungseinrichtung und somit relativ zu dem bezüglich der Führungseinrichtung feststehenden Werkzeugelement bewegt wird.

Günstigerweise kann vorgesehen sein, dass ein erstes relativ zu der Führungseinrichtung bewegbares Werkzeugelement mit einem bezüglich der Führungseinrichtung feststehenden Werkzeugelement ein erstes und ein zweites relativ zu der Führungseinrichtung bewegbares Werkzeugelement mit demselben bezüglich der Führungseinrichtung feststehenden Werkzeugelement ein zweites Werkzeug bilden. Durch die Doppelfunktion des bezüglich der Führungseinrichtung feststehenden Werkzeugelements, welches einerseits als Werkzeugelement für das erste Werkzeug und andererseits als Werkzeugelement für das zweite Werkzeug dient, ist ein besonders einfacher Aufbau des chirurgischen Instruments möglich. Ferner kann insbesondere bei der Beaufschlagung mindestens eines der Werkzeuge des chirurgischen Instruments mit Strom vorgesehen sein, dass das bezüglich der Führungseinrichtung feststehende Werkzeugelement stets mit einer elektrischen Leitung zur Zuführung von Strom verbunden ist, so dass zur wahlweisen Beaufschlagung des einen oder des anderen Werkzeugs des chirurgischen Instruments mit Strom jeweils lediglich zwischen einem Beaufschlagen des einen relativ zu der Führungseinrichtung bewegbaren Werkzeugelements und einem Beaufschlagen des anderen relativ zu der Führungseinrichtung bewegbaren Werkzeugelements umgeschalten werden muss.

Günstig ist es, wenn mindestens eines der mindestens zwei Werkzeugelemente bezüglich der Führungseinrichtung schwenkbar ist.

Insbesondere kann vorgesehen sein, dass ein erstes Werkzeugelement und ein zweites Werkzeugelement in einander entgegengesetzte Richtungen schwenkbar an der Führungseinrichtung gelagert sind.

Eine erste Achse, um welche beispielsweise ein erstes Werkzeugelement schwenkbar an der Führungseinrichtung gelagert ist, ist vorzugsweise von einer zweiten Achse, um welche beispielsweise ein zweites Werkzeugelement schwenkbar an der Führungseinrichtung gelagert ist, verschieden.

Ein einfacher Aufbau des chirurgischen Instruments ist insbesondere dann möglich, wenn die erste Achse parallel zur zweiten Achse ausgerichtet ist.

Grundsätzlich kann vorgesehen sein, dass das chirurgische Instrument aus einem elektrisch isolierenden Material gebildet ist, so dass mittels des chirurgischen Instruments kein Strom auf einen Körper einer zu operierenden Person übertragen werden kann. Um jedoch gezielt einen Strom von dem chirurgischen Instrument auf zu operierende Körperteile übertragen zu können, kann jedoch vorgesehen sein, dass mindestens eines der mindestens zwei Werkzeugelemente zumindest abschnittsweise elektrisch leitend ausgebildet ist. Auf diese Weise kann zumindest direkt an der Stelle, an welcher das mindestens eine der mindestens zwei Werkzeugelemente des chirurgischen Instruments angreift, ein elektrischer Strom auf das zu operierende Körperteil übertragen werden.

Bei einer Ausgestaltung der Erfindung ist vorgesehen, dass das chirurgische Instrument mindestens ein Isolierelement zum elektrischen Isolieren der Kraftübertragungseinrichtung, der Führungseinrichtung, mindestens eines Kopplungselements und/oder mindestens eines Werkzeugelements umfasst. Insbesondere ist es hierbei notwendig, bei einer bipolaren Beaufschlagung eines Werkzeugs des chirurgischen Instruments die zwei hierfür notwendigen elektrischen Leitungen, welche beispielsweise entlang der Führungseinrichtung verlaufen oder durch die Führungseinrichtung und/oder die Kraftübertragungseinrichtung gebildet sind, elektrisch voneinander zu isolieren.

Einerseits kann vorgesehen sein, dass mindestens ein Isolierelement als eine Beschichtung ausgebildet ist. Alternativ oder ergänzend hierzu kann vorgesehen sein, dass das mindestens eine Isolierelement als ein zusätzliches Bauteil des chirurgischen Instruments ausgebildet ist.

Das mindestens eine Isolierelement ist hierbei insbesondere aus einem elektrisch isolierenden Material, insbesondere einem Keramikwerkstoff, gebildet.

Bei dem erfindungsgemäßen chirurgischen Instrument kann die Kraftübertragungseinrichtung vorteilhafterweise sowohl zur mechanischen Umschaltung zwischen mindestens zwei Werkzeugelementen als auch zur elektrischen Umschaltung zwischen mindestens zwei Werkzeugelementen dienen. Auf diese Weise können grundsätzlich wahlweise die mindestens zwei Werkzeugelemente mit elektrischem Strom angesteuert und somit bipolar betrieben werden. Mindestens zwei Stromführungen für die mindestens zwei Werkzeugelemente und eine gegenseitige Isolation werden vorzugsweise durch ein einziges Element, nämlich die Kraftübertragungseinrichtung, ersetzt. Diese Kraftübertragungseinrichtung kann daher massiver ausgelegt werden, so dass eine höhere Stabilität des chirurgischen Instruments im Betrieb und somit eine geringere elastische Verformung und eine bessere Taktilität erreicht werden.

Der Aufbau des Handgriffs des chirurgischen Instruments wird hierdurch vorzugsweise vereinfacht. Dies ermöglicht ein leichteres und schlankeres Handgriffdesign.

Ferner kann bei dem erfindungsgemäßen chirurgischen Instrument vorgesehen sein, dass auf Grund von geringeren Toleranzunterschieden und geringerer elastischer Verformung im Umschaltbereich durch die Verwendung einer einzigen Kraftübertragungseinrichtung eine exaktere Umschaltung zwischen den Kopplungsstellungen der Kraftübertragungseinrichtung möglich ist.

Insbesondere um eine unerwünschte Betätigung des mindestens einen der mindestens zwei Werkzeugelemente, welches gerade nicht mit der Kraftübertragungseinrichtung gekoppelt ist, zu vermeiden, kann vorgesehen sein, dass das chirurgische Instrument eine Verriegelungsvorrichtung zur Verriegelung des gerade nicht benutzten mindestens einen Werkzeugelements umfasst.

Vorzugsweise umfasst die Verriegelungseinrichtung zur Verriegelung des gerade nicht benutzten mindestens einen Werkzeugelements mindestens ein Blockierelement zum Blockieren einer Bewegung, insbesondere einer Bewegung in der Kraftübertragungsrichtung, des mindestens einen nicht mit der Kraftübertragungseinrichtung gekoppelten Werkzeugelements umfasst.

Günstig ist es, wenn das mindestens eine Blockierelement im Bereich des distalen Endes der Kraftübertragungseinrichtung angeordnet ist. Vorzugsweise ist das Blockierelement am Instrument bewegbar gehalten.

Insbesondere kann vorgesehen sein, dass das mindestens eine Blockierelement im Bereich des distalen Endes der Kraftübertragungseinrichtung mit der Kraftübertragungseinrichtung zusammenwirkt. Vorzugsweise ist das Blockierelement derart gehalten, dass es infolge einer Bewegung der Kraftübertragungseinrichtung automatisch mitbewegt wird. Beispielsweise kann die Kraftübertragungseinrichtung mindestens teilweise als Mitnehmer ausgebildet sein, welcher am Blockierelement anliegt und eine Zwangsbewegung des Blockierelements zum Beispiel infolge einer Drehung der Kraftübertragungseinrichtung um eine Längsachse der Führungseinrichtung bewirkt.

Ferner kann vorgesehen sein, dass das mindestens eine Blockierelement im Bereich des distalen Endes der Führungseinrichtung angeordnet ist.

Das mindestens eine Blockierelement ist vorzugsweise an der Führungseinrichtung geführt bewegbar gehalten. Beispielsweise kann eine teilweise an der Führungseinrichtung und teilweise am Blockierelement ausgebildet Nut-Feder-Führung vorgesehen sein, um eine Bewegung des Blockierelements und der Führungseinrichtung relativ zueinander zu führen.

Vorzugsweise ist das mindestens eine Blockierelement um eine Drehachse, insbesondere um eine Drehachse der Führungseinrichtung im Bereich des distalen Endes der Führungseinrichtung, beispielsweise um die Mittelachse der Führungseinrichtung, drehbar.

Insbesondere um unerwünschte elektrische Kontakte zu vermeiden, kann vorgesehen sein, dass das mindestens eine Blockierelement zumindest teilweise eine nichtleitende Oberfläche aufweist. Vorzugsweise ist die gesamte Oberfläche des Blockierelements nichtleitend, das heißt elektrisch isolierend ausgebildet, beispielsweise durch Vorsehen einer isolierenden Beschichtung. Ferner kann vorgesehen sein, dass das mindestens eine Blockierelement zumindest teilweise, vorzugsweise vollständig, aus einem nichtleitenden, das heißt elektrisch isolierenden, Material, beispielsweise aus einem keramischen Material oder einem Kunststoff, gebildet ist

Besonders günstig ist es, wenn das mindestens eine Blockierelement mittels einer Betätigungseinrichtung, beispielsweise mittels der Betätigungseinrichtung zum Wechseln einer Kopplungsstellung der Kopplungseinrichtung, betätigbar ist. Das mindestens eine Blockierelement ist hierbei vorzugsweise aus einer ersten Blockierstellung, in welcher die Bewegung des ersten bewegbaren Werkzeugelements blockiert ist, in eine zweite Blockierstellung bringbar, in welcher die Bewegung des zweiten bewegbaren Werkzeugelements blockiert ist.

Günstig ist es, wenn das mindestens eine Blockierelement so mit der Kraftübertragungseinrichtung zusammenwirkt, dass bei einer Drehung der Kraftübertragungseinrichtung relativ zu den bewegbaren Werkzeugelementen zum Wechseln einer Kopplungsstellung zugleich eine Drehung des mindestens einen Blockierelements erfolgt, um das mindestens eine Blockierelement von einer ersten Blockierstellung in eine weitere Blockierstellung, beispielsweise von der ersten Blockierstellung in die zweite Blockierstellung, zu bringen.

Mittels des mindestens einen Blockierelements ist vorzugsweise mindestens ein bewegbares Werkzeugelement in einer geschlossenen Stellung blockierbar.

Alternativ hierzu kann jedoch auch vorgesehen sein, dass mittels des mindestens einen Blockierelements mindestens ein Werkzeugelement in einer offenen Stellung blockierbar ist.

Die Verwendung mindestens eines Blockierelements zum Blockieren einer Bewegung mindestens eines nicht mit der Kraftübertragungseinrichtung gekoppelten Werkzeugelements kann insbesondere den Vorteil haben, dass eine unerwünschte Betätigung des nicht mit der Kraftübertragungseinrichtung gekoppelten Werkzeugelements bei einer Verwendung des chirurgischen Instruments verhindert wird. Somit ist eine besonders sichere Handhabung des chirurgischen Instruments möglich.

Weitere Merkmale und Vorteile der Erfindung sind Gegenstand der nachfolgenden Beschreibung und der zeichnerischen Darstellung eines Ausführungsbeispiels. In den Figuren zeigen:
- Figur 1:: eine schematische perspektivische Darstellung eines chirurgischen Instruments;
- Figur 2:: eine schematische perspektivische Darstellung eines distalen Endes einer Führungseinrichtung des chirurgischen Instruments, mit einer in der Führungseinrichtung geführten Kraftübertragungseinrichtung, an dem distalen Ende der Führungseinrichtung angeordneten Werkzeugelementen und einer Kopplungseinrichtung zur Kopplung der Kraftübertragungseinrichtung mit den Werkzeugelementen;
- Figur 3:: Eine schematische perspektivische Darstellung eines horizontalen Schnitts durch die Kraftübertragungseinrichtung, die Kopplungseinrichtung und die Werkzeugelemente aus Figur 2 entlang der Linie 3-3 in Figur 2;
- Figur 4:: eine schematische Seitenansicht der Kraftübertragungseinrichtung, der Kopplungseinrichtung und zweier Werkzeugelemente, wobei ein erstes bewegbares Werkzeugelement in einer Offenstellung angeordnet ist;
- Figur 5:: eine schematische perspektivische Darstellung der Kopplungseinrichtung und der Werkzeugelemente, mit Blick auf ein zweites bewegbares Werkzeugelement;
- Figur 6:: eine vergrößerte Darstellung eines distalen Endes der Kraftübertragungseinrichtung und eines der Kraftübertragungseinrichtung zugewandten Endes der Kopplungseinrichtung, wobei zwei Kopplungselemente der Kopplungseinrichtung entlang der Linie 6-6 in Figur 5 in horizontaler Richtung geschnitten sind und die Kraftübertragungseinrichtung in einer Zwischenstellung zwischen zwei Kopplungsstellungen angeordnet ist;
- Figur 7:: eine schematische perspektivische Darstellung der Kraftübertragungseinrichtung, der Kopplungseinrichtung und der Werkzeugelemente, wobei die Kopplungseinrichtung mit einem dem zweiten bewegbaren Werkzeug zugeordneten zweiten Kopplungselement gekoppelt ist;
- Figur 8:: eine schematische perspektivische Darstellung der Kraftübertragungseinrichtung, der Kopplungseinrichtung und der Werkzeugelemente mit Blick auf das zweite bewegbare Werkzeugelement, wobei die Kraftübertragungseinrichtung mit dem dem zweiten bewegbaren Werkzeugelement zugeordneten Kopplungselement gekoppelt ist;
- Figur 9:: eine schematische Seitenansicht der Kraftübertragungseinrichtung, der Kopplungseinrichtung und der Werkzeugelemente mit Blick auf das zweite bewegbare Werkzeugelement, welches in einer Offenstellung angeordnet ist;
- Figur 10:: eine erste Explosionsdarstellung der Werkzeugelemente und der Kopplungseinrichtung; und
- Figur 11:: eine zweite Explosionsdarstellung der Werkzeugelemente und der Kopplungseinrichtung.

Gleiche oder funktional äquivalente Merkmale sind in sämtlichen Figuren mit denselben Bezugszeichen versehen.

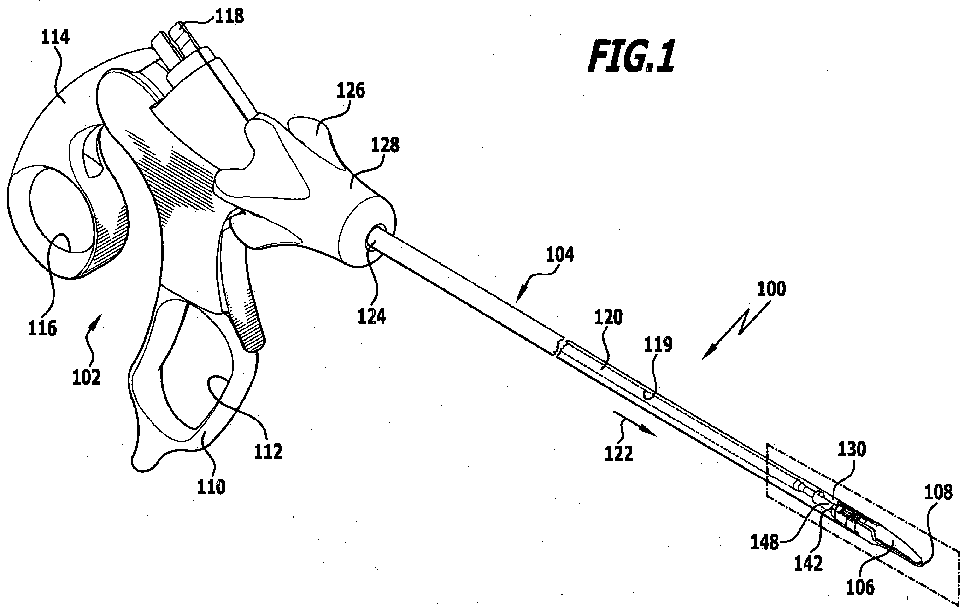

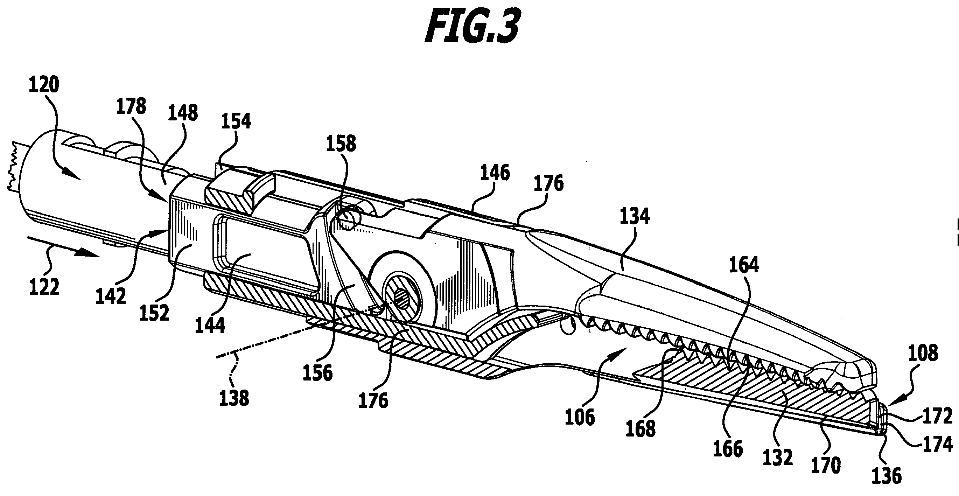

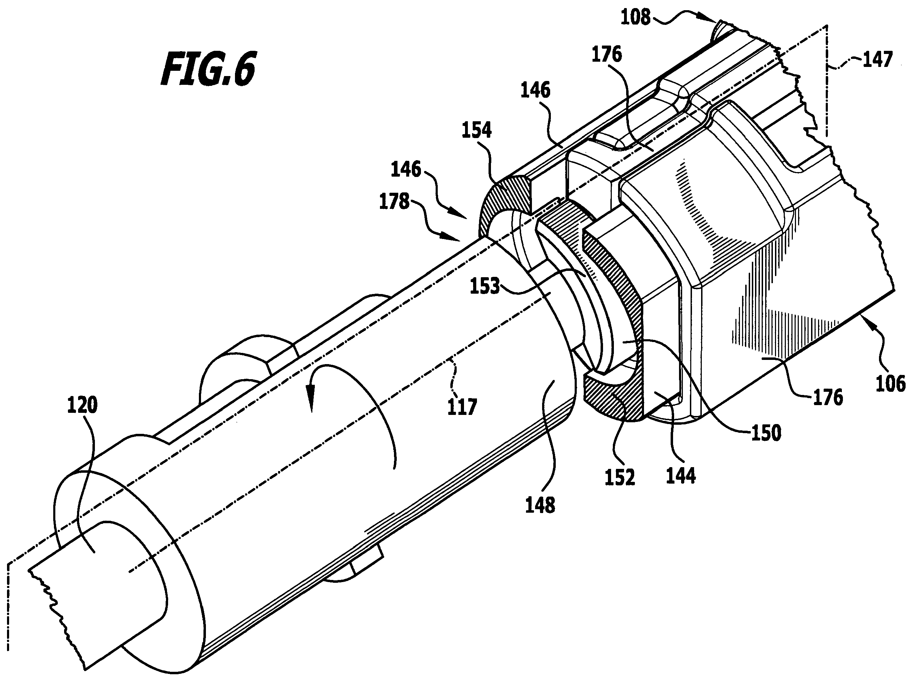

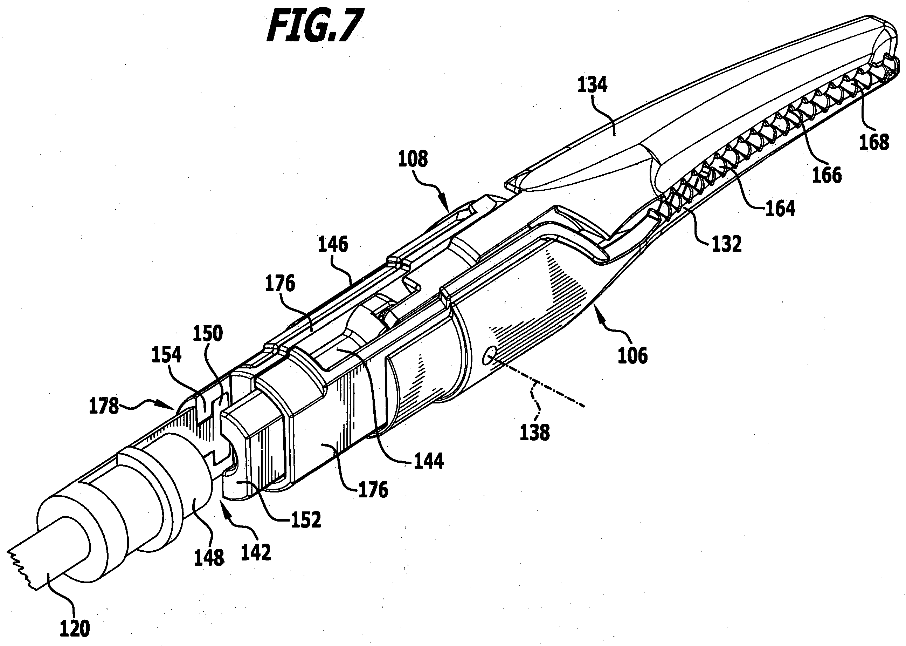

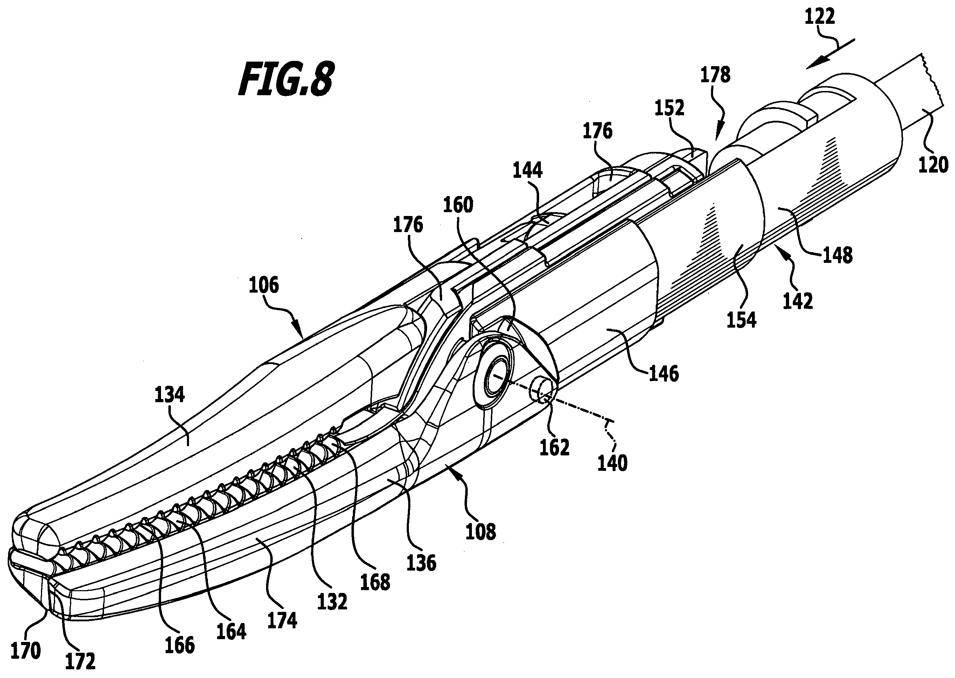

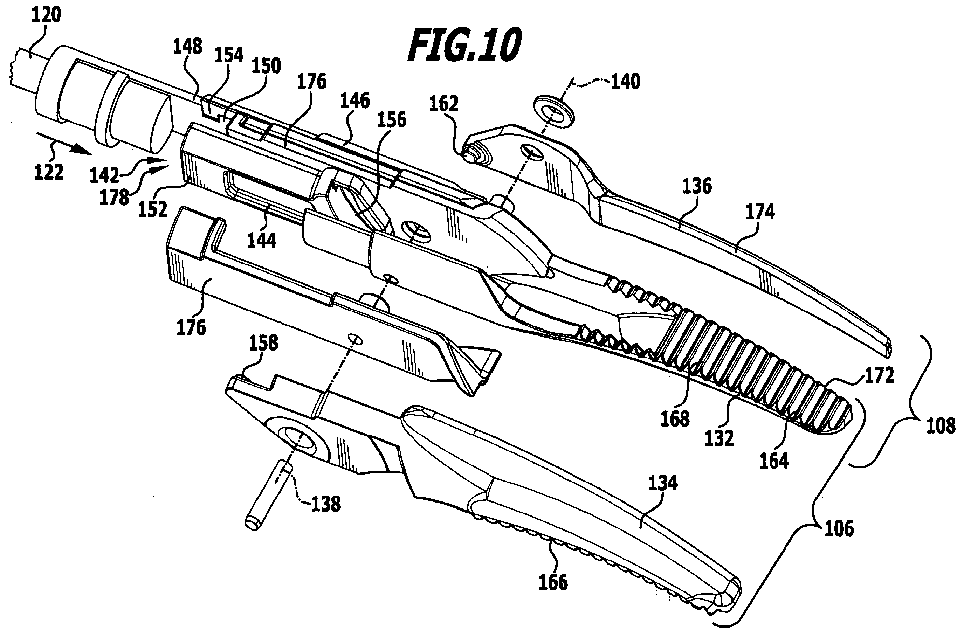

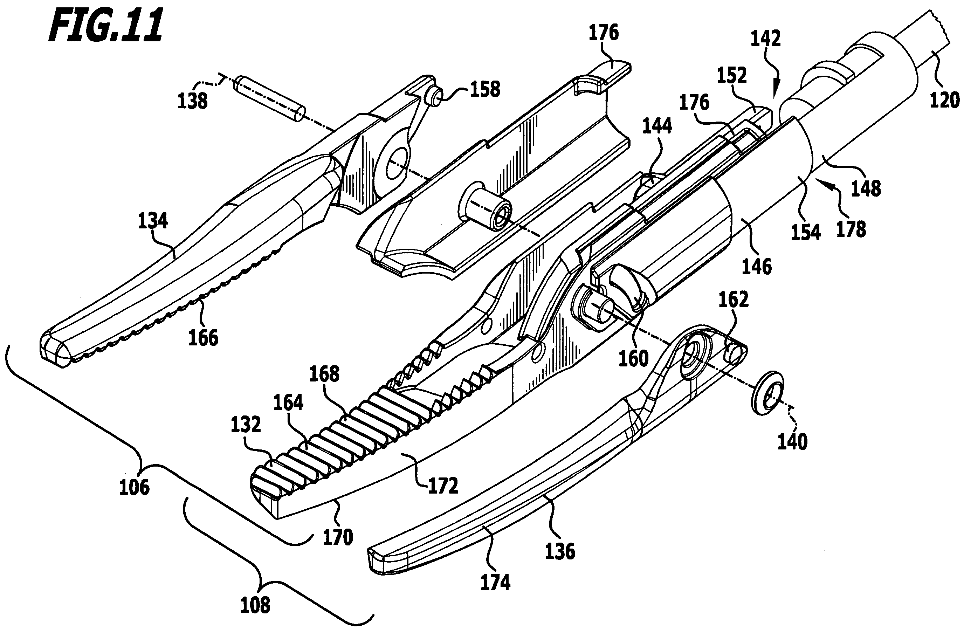

Ein beispielhaft in den Figuren 1 bis 11 dargestelltes chirurgisches Instrument 100 umfasst einen Handgriff 102, eine Führungseinrichtung 104, ein erstes Werkzeug 106 und ein zweites Werkzeug 108.

Der Handgriff 102 umfasst eine feststehende Branche 110 mit einer Fingeröffnung 112 und eine bewegbare Branche 114 mit einer Fingeröffnung 116. An dem Handgriff 102 kann beispielsweise ein Chirurg angreifen und durch eine Relativbewegung der bewegbaren Branche 114 zu der feststehenden Branche 110 das erste Werkzeug 106 oder das zweite Werkzeug 108 betätigen.

Ferner umfasst der Handgriff 102 Anschlüsse 118 zum Anlegen einer Spannung, welche mittels elektrischer Leitungen mit dem ersten Werkzeug 106 und/oder dem zweiten Werkzeug 108 verbunden werden können.

Die Führungseinrichtung 104 ist im Wesentlichen rotationssymmetrisch um eine Mittelachse 117 der Führungseinrichtung 104 ausgebildet und umfasst einen zylindrischen Hohlraum 119. Die Führungseinrichtung 104 ist somit als ein langgestreckter Schaft ausgebildet.

Der Handgriff 102 ist mit der Führungseinrichtung 104 und einer in dem.zylindrischen Hohlraum 119 der Führungseinrichtung 104 geführten Kraftübertragungseinrichtung 120 verbunden, wobei eine Bewegung in einer Kraftübertragungsrichtung 122 der Kraftübertragungseinrichtung 120 relativ zu der Führungseinrichtung 104 und/oder eine Drehbewegung der Kraftübertragungseinrichtung 120 relativ zu der Führungseinrichtung 104 gewährleistet ist.

Insbesondere ist hierbei vorgesehen, dass die Kraftübertragungseinrichtung 120 drehfest an dem Handgriff 102 angeordnet ist und dass die Führungseinrichtung 104 um eine parallel zu der Kraftübertragungsrichtung 122 verlaufende Achse drehbar an dem Handgriff 102 gelagert ist.

Zum Drehen der Führungseinrichtung 104 um die parallel zu der Kraftübertragungsrichtung 122 ausgerichtete Achse ist an einem dem Handgriff 102 zugewandten, proximalen Ende 124 der Führungseinrichtung 104 eine beispielsweise als Drehelement 126 ausgebildete Betätigungseinrichtung 128 vorgesehen. Mittels der Betätigungseinrichtung 128 kann ein Chirurg, welcher das chirurgische Instrument 100 bedient, die Führungseinrichtung 104 besonders einfach um die parallel zu der Kraftübertragungsrichtung 122 ausgerichtete Achse drehen. An einem dem Handgriff 102 abgewandten, distalen Ende 130 der Führungseinrichtung 104 sind das erste Werkzeug 106 und das zweite Werkzeug 108 angeordnet.

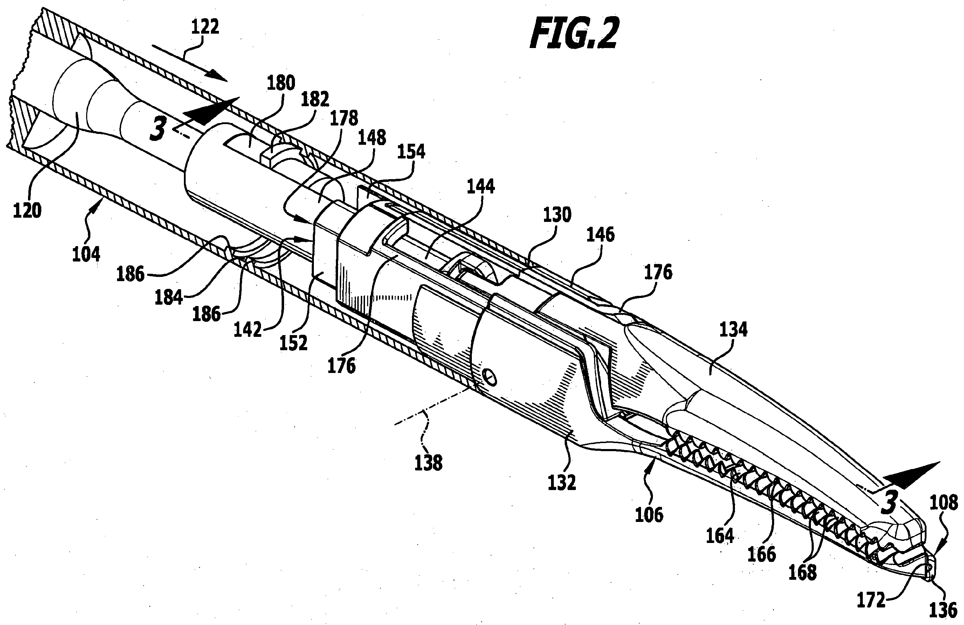

Wie insbesondere Figur 2 zu entnehmen ist, umfasst das chirurgische Instrument 100 ein bezüglich der Führungseinrichtung 104 feststehendes Werkzeugelement 132, ein schwenkbar an dem Führungselement 104 gelagertes erstes bewegbares Werkzeugelement 134 und ein schwenkbar an der Führungseinrichtung 104 gelagertes zweites bewegbares Werkzeugelement 136. Das feststehende Werkzeugelement 132 bildet zusammen mit dem ersten bewegbaren Werkzeugelement 134 das erste Werkzeug 106. Zusammen mit dem zweiten bewegbaren Werkzeugelement 136 bildet das feststehende Werkzeugelement 132 ferner das zweite Werkzeug 108.

Das erste bewegbare Werkzeugelement 134 ist um eine erste Achse 138 schwenkbar an der Führungseinrichtung 104 gelagert, wobei die erste Achse 138 senkrecht zu der Kraftübertragungsrichtung 122 ausgerichtet ist.

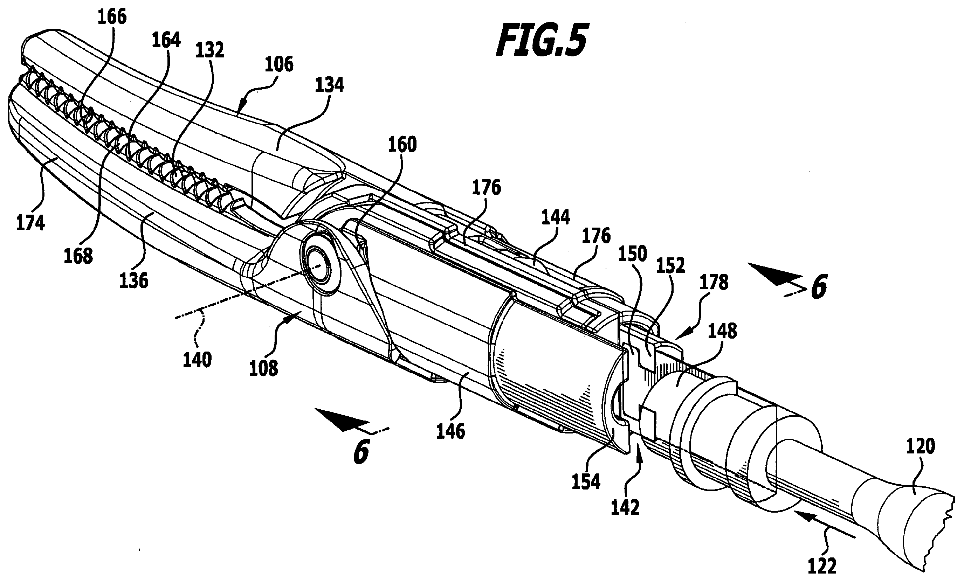

Das zweite bewegbare Werkzeugelement 136 ist um eine zweite Achse 140 schwenkbar an der Führungseinrichtung 104 gelagert (siehe Figur 5), wobei die zweite Achse 140 ebenfalls senkrecht zu der Kraftübertragungsrichtung 122 ausgerichtet ist, jedoch nicht mit der ersten Achse 138 zusammenfällt. Die erste Achse 138 und die zweite Achse 140 sind jedoch vorzugsweise parallel zueinander ausgerichtet.

Das chirurgische Instrument 100 umfasst ferner eine Kopplungseinrichtung 142 zum wahlweisen Koppeln der Kraftübertragungseinrichtung 120 mit dem ersten bewegbaren Werkzeugelement 134 oder dem zweiten bewegbaren Werkzeugelement 136. Hierzu umfasst die Kopplungseinrichtung 142 zwei Kopplungselemente, nämlich ein dem ersten bewegbaren Werkzeugelement 134 zugeordnetes erstes Kopplungselement 144 und ein dem zweiten bewegbaren Werkzeugelement 136 zugeordnetes zweites Kopplungselement 146. Die Kopplungselemente 144, 146 sind auf einander gegenüberliegenden Seiten einer durch die Mittelachse 117 der Führungseinrichtung 104 verlaufenden Längsmittelebene 147 der Führungseinrichtung 104 angeordnet.

Wie insbesondere Figur 5 zu entnehmen ist, umfasst die Kraftübertragungseinrichtung 120 an einem distalen Ende 148 der Kraftübertragungseinrichtung 120 ein Kopplungsglied 150, welches in der Kraftübertragungsrichtung 122 formschlüssig mit einem Kopplungsglied 152 des dem ersten bewegbaren Werkzeugelement 134 zugeordneten ersten Kopplungselements 144 verbindbar ist.

In einer beispielsweise in Figur 5 dargestellten Schließstellung des ersten bewegbaren Werkzeugelements 134 und des zweiten bewegbaren Werkzeugelements 136 ist das Kopplungsglied 152 des dem ersten bewegbaren Werkzeugelement 134 zugeordneten ersten Kopplungselements 144 einem Kopplungsglied 154 des dem zweiten bewegbaren Werkzeugelement 136 zugeordneten zweiten Kopplungselements 146 gegenüberliegend angeordnet. Auch dieses Kopplungsglied 154 des dem zweiten bewegbaren Werkzeugelement 136 zugeordneten zweiten Kopplungselements 146 ist so ausgebildet, dass es mit dem Kopplungsglied 150 der Kraftübertragungseinrichtung 120 in der Kraftübertragungsrichtung 122 formschlüssig verbindbar ist.

Die Kopplungseinrichtung 142 umfasst mindestens eine beispielsweise als Einführschräge ausgebildete Kopplungshilfe 153 zur Vereinfachung einer Kopplung der Kopplungsglieder 150, 152, 154.

Bei der in Figur 5 dargestellten Kopplungsstellung, das heißt bei einer Anordnung der Kraftübertragungseinrichtung 120 und der Kopplungseinrichtung 142 derart, dass das Kopplungsglied 150 der Kraftübertragungseinrichtung 120 mit dem Kopplungsglied 152 des dem ersten bewegbaren Werkzeugelement 134 zugeordneten ersten Kopplungselements 144 in Eingriff ist, ist mittels der Kraftübertragungseinrichtung 120 eine Kraft auf das erste bewegbare Werkzeugelement 134 übertragbar, da das erste Kopplungselement 144 die auf dasselbe übertragene Kraft an das erste bewegbare Werkzeugelement 134 weitergibt.

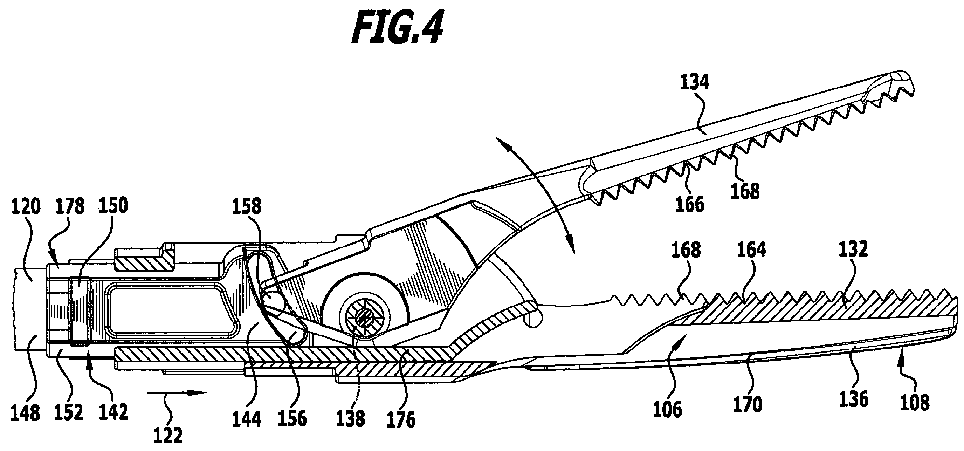

Wie Figur 4 zu entnehmen ist, umfasst das erste Kopplungselement 144 hierzu eine Führungsbahn 156, in welche ein Führungszapfen 158 des ersten bewegbaren Werkzeugelements 134 eingreift. Durch eine Bewegung des ersten Kopplungselements 144 mittels der Kraftübertragungseinrichtung 120 wird der Führungszapfen 158 des ersten bewegbaren Werkzeugelements 134 entlang der Führungsbahn 156 verschoben, so dass das erste bewegbare Werkzeugelement 134 um die erste Achse 138 verschwenkt wird. Die Bewegung der Kraftübertragungseinrichtung 120 entlang der Kraftübertragungsrichtung 122 wird somit mittels des ersten Kopplungselements 144 in eine Schwenkbewegung des ersten bewegbaren Werkzeugelements 134 umgewandelt.

Die Kopplungseinrichtung 142 und die Werkzeuge 106, 108 sind bezüglich einer parallel zu der Kraftübertragungsrichtung 122 verlaufenden Achse drehfest an der Führungseinrichtung 104 angeordnet, so dass bei einer Drehung der Kraftübertragungseinrichtung 120 relativ zu der Führungseinrichtung 104 um eine Achse parallel zu der Kraftübertragungsrichtung 122 auch eine Drehbewegung der Kraftübertragungseinrichtung 120 relativ zu der Kopplungseinrichtung 142 durchgeführt wird.

Wie bereits ausgeführt, kann hierzu entweder die Kraftübertragungseinrichtung 120 relativ zu dem Handgriff 102 gedreht werden, wenn die Führungseinrichtung 104 drehfest mit dem Handgriff 102 verbunden ist. Alternativ hierzu kann jedoch auch die Führungseinrichtung 104 zusammen mit der Kopplungseinrichtung 142 und den Werkzeugen 106, 108 gedreht werden, wenn die Kraftübertragungseinrichtung 120 drehfest an dem Handgriff 102 angeordnet ist. Durch eine Drehung der Kraftübertragungseinrichtung 120 relativ zu der Kopplungseinrichtung 142 ist das Kopplungsglied 150 der Kraftübertragungseinrichtung 120 mit dem Kopplungsglied 152 des dem ersten bewegbaren Werkzeugelement 134 zugeordneten ersten Kopplungselements 144 außer Eingriff und mit dem Kopplungsglied 154 des dem zweiten bewegbaren Werkzeugelement 136 zugeordneten zweiten Kopplungselements 146 in Eingriff bringbar.

Auf diese Weise kann die Kraftübertragungseinrichtung 120 wahlweise mit dem ersten Kopplungselement 144 und somit mit dem ersten bewegbaren Werkzeugelement 134 oder mit dem zweiten Kopplungselement 146 und somit mit dem zweiten bewegbaren Werkzeugelement 136 gekoppelt werden.

Das zweite Kopplungselement 146 umfasst eine Führungsbahn 160, in welcher ein Führungszapfen 162 des zweiten bewegbaren Werkzeugelements 136 geführt ist. Der Funktion des ersten Kopplungselements 144 und des ersten bewegbaren Werkzeugelements 134 entsprechend, kann mittels des zweiten Kopplungselements 146 eine von der Kraftübertragungseinrichtung 120 auf das zweite Kopplungselement 146 übertragene Bewegung in der Kraftübertragungsrichtung 122 mittels der Führungsbahn 160 und des Führungszapfens 162 in eine Schwenkbewegung des zweiten bewegbaren Werkzeugelements 136 umgewandelt werden.

Das erste bewegbare Werkzeugelement 134 und das zweite bewegbare Werkzeugelement 136 greifen vorzugsweise an einander gegenüberliegenden Seiten des bezüglich der Führungseinrichtung 104 feststehenden Werkzeugelements 132 an.

Eine dem ersten bewegbaren Werkzeugelement 134 zugewandte Oberseite 164 des feststehenden Werkzeugelements 132 und eine dem feststehenden Werkzeugelement 132 zugewandte Unterseite 166 des ersten bewegbaren Werkzeugelements 134 weisen spitze Vorsprünge 168 auf, welche ein besonders sicheres Festklemmen eines zwischen das erste bewegbare Werkzeugelement 134 und das feststehende Werkzeugelement 132 geklemmten Objekts ermöglichen (siehe Figur 4).

Das durch das erste bewegbare Werkzeugelement 134 und das feststehende Werkzeugelement 132 gebildete Werkzeug 106 ist somit als Klemmwerkzeug ausgebildet.

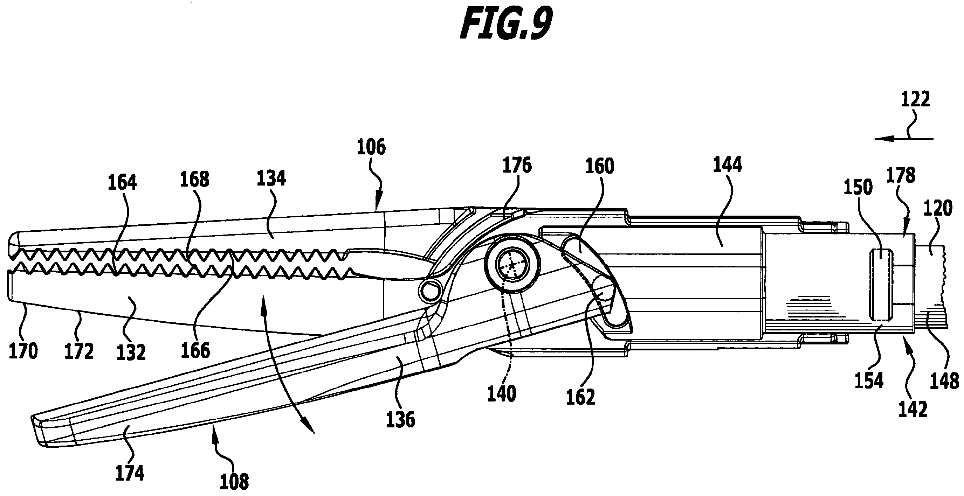

Eine der Oberseite 164 des feststehenden Werkzeugelements 132 abgewandte Unterseite 170 des feststehenden Werkzeugelements 132 ist als Schneidkante 172 ausgebildet, welche mit dem als Schneidmesser 174 ausgebildeten zweiten bewegbaren Werkzeugelement 136 zusammenwirkt, so dass das durch das zweite bewegbare Werkzeugelement 136 und das feststehende Werkzeugelement 132 gebildete zweite Werkzeug 108 als Schneidwerkzeug ausgebildet ist (siehe Figur 9).

Das chirurgische Instrument 100 ist somit wahlweise als Klemmwerkzeug oder als Schneidwerkzeug verwendbar. Zusätzlich zu der Klemmfunktion und der Schneidefunktion des chirurgischen Instruments 100 kann mittels des chirurgischen Instruments 100 ferner ein Strom auf ein zu bearbeitendes Objekt übertragen werden. Hierzu sind vorzugsweise die bewegbaren Werkzeugelemente 134, 136 einerseits und das feststehende Werkzeugelement 132 andererseits mit bipolarem Strom beaufschlagbar.

Bei der in den Figuren 1 bis 11 dargestellten Ausführungsform eines chirurgischen Instruments 100 ist jedoch keine Beaufschlagung des zweiten bewegbaren Werkzeugelements 136 mit Strom vorgesehen. Vielmehr ist lediglich das erste bewegbare Werkzeugelement 134 zusammen mit dem feststehenden Werkzeugelement 132 mit Strom beaufschlagbar.

Hierzu sind die Führungseinrichtung 104 und die Kraftübertragungseinrichtung 120 zumindest teilweise elektrisch leitend ausgebildet, wobei die Führungseinrichtung 104 und die Kraftübertragungseinrichtung 120 elektrisch gegeneinander isoliert sind. Zum Übertragen eines bipolaren Stroms zu dem ersten Werkzeug 106 ist die Verbindung zwischen dem feststehenden Werkzeugelement 132 und der Führungseinrichtung 104 elektrisch leitend. Die Führungseinrichtung 104 wiederum ist mit einem der Anschlüsse 118 elektrisch verbunden. Der andere der Anschlüsse 118 ist mit der Kraftübertragungseinrichtung 120 elektrisch verbunden, welche an ihrem distalen Ende 148 mit dem elektrisch leitenden ersten Kopplungselement 144 in Eingriff bringbar ist. Da die Verbindung zwischen dem ersten Kopplungselement 144 und der Kraftübertragungseinrichtung 120 und die Verbindung zwischen dem ersten Kopplungselement 144 und dem ersten bewegbaren Werkzeugelement 134 die Übertragung eines elektrischen Strom ermöglichen, kann von der Kraftübertragungseinrichtung 120 auf das erste bewegbare Werkzeugelement 134 ein Strom übertragen werden. Um einen Kurzschluss zwischen der Kraftübertragungseinrichtung 120, dem ersten Kopplungselement 144 und dem ersten bewegbaren Werkzeugelement 134 einerseits und der Führungseinrichtung 104 und dem bezüglich der Führungseinrichtung 104 feststehenden Werkzeugelement 132 zu vermeiden, sind an dem distalen Ende 130 der Führungseinrichtung 104 zwei Isolierelemente 176 vorgesehen, welche das erste Kopplungselement 144 und das erste bewegbare Werkzeugelement 134 räumlich von der Führungseinrichtung 104 und dem feststehenden Werkzeugelement 132 trennen und aus einem elektrisch isolierenden Material gebildet sind.

Die Kopplungseinrichtung 142 dient grundsätzlich auch als elektrische Umschalteinrichtung 178, mit welcher wahlweise zwischen einem Beaufschlagen des ersten bewegbaren Werkzeugelements 134 oder des zweiten bewegbaren Werkzeugelements 136 mit Strom umgeschaltet werden kann.

Bei der in den Figuren 1 bis 11 dargestellten Ausführungsform ist jedoch vorgesehen, dass von der Kraftübertragungseinrichtung 120 lediglich auf das erste Kopplungselement 144 ein Strom übertragbar ist, um einen Kurzschluss an dem gegenüber der Führungseinrichtung 104 nicht isolierten zweiten bewegbaren Werkzeugelement 136 zu vermeiden. Hierzu kann beispielsweise das Kopplungselement 146 aus einem elektrisch isolierenden Material gebildet oder mit einer elektrisch isolierenden Beschichtung versehen sein.

Wie insbesondere in Figur 2 erkennbar, umfasst das chirurgische Instrument 100 ein Blockierelement 180, welches mittels eines ringabschnittsförmigen Vorsprungs 182 des Blockierelements 180 an der Innenseite der Führungseinrichtung 104 geführt ist. Die Führungseinrichtung 104 weist hierzu eine ringförmige Nut 184 auf, in welche sich der Vorsprung 182 des Blockierelements 180 hinein erstreckt.

Die Nut 184 der Führungseinrichtung 104 verläuft in einer Ebene senkrecht zu der Kraftübertragungsrichtung 122 der Kraftübertragungseinrichtung 120, so dass das Blockierelement 180 nicht in der Kraftübertragungsrichtung 122 verschoben, jedoch um die Mittelachse 117 gedreht werden kann.

Die Nut 184 der Führungseinrichtung 104 ist mittels zweier ringförmiger Vorsprünge 186 der Führungseinrichtung 104 gebildet. In einer nicht dargestellten, weiteren Ausführungsform kann die Nut 184 jedoch auch beispielsweise lediglich durch eine ringförmige Ausnehmung in einer Wand der Führungseinrichtung 104 gebildet sein.

Das Blockierelement 180 liegt im montierten Zustand des chirurgischen Instruments 100 an der Kraftübertragungseinrichtung 120 an und ist mittels einer Drehung der Kraftübertragungseinrichtung 120 um die Mittelachse 117 der Führungseinrichtung 104 drehbar. Hierdurch kann das Blockierelement 180 aus einer Stellung, in welcher die Kraftübertragungseinrichtung 120 beispielsweise mit dem ersten bewegbaren Werkzeugelement 134 gekoppelt ist und das Blockierelement 180 eine Bewegung des zweiten bewegbaren Werkzeugelements 136 blockiert, in eine Stellung gebracht werden, in welcher die Kraftübertragungseinrichtung 120 mit dem zweiten bewegbaren Werkzeugelement 136 gekoppelt ist und die Blockiereinrichtung 180 eine Bewegung des ersten bewegbaren Werkzeugelements 134 blockiert.

Zur elektrischen Isolation von den angrenzenden Bauteilen des chirurgischen Instruments 100 kann vorgesehen sein, dass das Blockierelement 180 zumindest in dem Bereich, in welchem es mit der Führungseinrichtung 104 und/oder mit der Kraftübertragungseinrichtung 120 in Kontakt kommt, eine elektrisch nichtleitende Oberfläche aufweist. Ferner kann vorgesehen sein, dass das Blockierelement 180, insbesondere vollständig, aus einem elektrisch isolierenden Material, beispielsweise aus einem keramischen Material, gebildet ist.

Das vorstehend beschriebene chirurgische Instrument 100 wird vorzugsweise wie folgt verwendet:
Vorbereitend wird an die Anschlüsse 118 eine elektrische Spannung angelegt, so dass das erste Werkzeug 106 mit bipolarem Strom versorgt werden kann.

Die eigentliche Benutzung des chirurgischen Instruments 100 erfolgt üblicherweise durch einen Chirurgen, welcher an dem Handgriff 102 angreift und durch die Betätigung der Betätigungseinrichtung 128 die Führungseinrichtung 104 samt der Kopplungseinrichtung 142 und der Werkzeuge 106, 108 relativ zu der Kraftübertragungseinrichtung 120 dreht, um das chirurgische Instrument 100 zur Betätigung des gewünschten Werkzeugs 106, 108 vorzubereiten.

Durch die Drehung der Führungseinrichtung 104 relativ zur der Kraftübertragungseinrichtung 120 wird die Kraftübertragungseinrichtung 120 wahlweise mit dem ersten bewegbaren Werkzeugelement 134 oder dem zweiten bewegbaren Werkzeugelement 136 gekoppelt. Insbesondere wird hierzu das Kopplungsglied 150 der Kraftübertragungseinrichtung 120 wahlweise mit dem Kopplungsglied 152 des dem ersten bewegbaren Werkzeugelement 134 zugeordneten ersten Kopplungselements 144 oder dem Kopplungsglied 154 des dem zweiten bewegbaren Werkzeugelement 136 zugeordneten zweiten Kopplungselements 146 in Eingriff gebracht. Das jeweils nicht mit der Kraftübertragungseinrichtung 120 gekoppelte Werkzeugelement 134, 136 wird dabei mittels des Blockierelements 180 vorzugsweise in der geschlossenen Stellung gehalten oder blockiert, so dass eine unerwünschte Bewegung des nicht mit der Kraftübertragungseinrichtung 120 gekoppelten Werkzeugelements 134, 136 vermieden wird. Durch eine Bewegung der bewegbaren Branche 114 des Handgriffs 102 relativ zur der feststehenden Branche 110 des Handgriffs 102 wird die Kraftübertragungseinrichtung 120 in der Kraftübertragungsrichtung 122 entlang der Führungseinrichtung 104 verschoben und damit eine Betätigungskraft von dem proximalen Ende 124 der Führungseinrichtung 104 zu dem distalen Ende 130 der Führungseinrichtung 104 übertragen. Die Bewegung der Kraftübertragungseinrichtung 120 in der Kraftübertragungsrichtung 122 wird mittels des an die Kraftübertragungseinrichtung 120 gekoppelten Kopplungselements, das heißt entweder des ersten Kopplungselements 144 oder des zweiten Kopplungselements 146, auf das zu bewegende bewegbare Werkzeugelement, das heißt entweder auf das erste bewegbare Werkzeugelement 134 oder auf das zweite bewegbare Werkzeugelement 136 übertragen, wobei die Bewegung der Kraftübertragungseinrichtung 120 in der Kraftübertragungsrichtung 122 mittels des ersten Kopplungselements 144 beziehungsweise mittels des zweiten Kopplungselements 146 in eine Schwenkbewegung des ersten bewegbaren Werkzeugelements 134 beziehungsweise eine Schwenkbewegung des zweiten bewegbaren Werkzeugelements 136 umgewandelt wird. Durch die Bewegung des ersten bewegbaren Werkzeugelements 134 kann das erste Werkzeug 106 in eine Offenstellung (siehe Figur 4) gebracht werden, in welcher zwischen das erste bewegbare Werkzeugelement 134 und das feststehende Werkzeugelement 132 ein mittels des ersten Werkzeugs 106 zu ergreifendes Objekt einlegbar ist. Beispielsweise kann mittels des ersten Werkzeugs 106 auf diese Weise eine Arterie ergriffen werden. Mittels des auf das erste Werkzeug 106 übertragbaren bipolaren Stroms, welcher vorzugsweise ein hochfrequenter (HF) Strom ist, kann die Arterie dann verschweißt werden.

Nach einem Öffnen des ersten Werkzeugs 106 und einem Entfernen der Arterie aus dem ersten Werkzeug 106 wird das erste Werkzeug 106 erneut geschlossen, so dass durch eine erneute Betätigung der Betätigungseinrichtung 128 die Kraftübertragungseinrichtung 120 erneut relativ zur der Führungseinrichtung 104 gedreht werden kann, um das Kopplungsglied 150 der Kraftübertragungseinrichtung 120 in einem nächsten Schritt mit dem Kopplungsglied 154 des dem zweiten bewegbaren Werkzeugelement 136 zugeordneten zweiten Kopplungselements 146 in Eingriff zu bringen.

Durch ein Verschieben der Kraftübertragungseinrichtung 120 in der Kraftübertragungsrichtung 122 relativ zu der Führungseinrichtung 104 mittels einer Bewegung der bewegbaren Branche 114 relativ zu der feststehenden Branche 110 des Handgriffs 102 kann das zweite Werkzeug 108 in eine Offenstellung gebracht werden, das heißt, es kann das zweite bewegbare Werkzeugelement 136 von dem feststehenden Werkzeugelement 132 weg geschwenkt werden, so dass beispielsweise die zuvor bearbeitete Arterie zwischen die Schneidkante 172 an der Unterseite 170 des feststehenden Werkzeugelements 132 und das als Schneidmesser 174 ausgebildete zweite bewegbare Werkzeugelement 136 eingelegt werden kann. Durch ein Zurückziehen der Kraftübertragungseinrichtung 120 entgegen der Kraftübertragungsrichtung 122 relativ zu der Führungseinrichtung 104 wird das zweite Werkzeug 108 geschlossen; das als Schneidmesser 174 ausgebildete zweite bewegbare Werkzeugelement 136 bewegt sich dabei auf die Schneidkante 172 an der Unterseite 170 des feststehenden Werkzeugelements 132 zu und durchtrennt die dazwischen angeordnete Arterie.

Dadurch, dass das chirurgische Instrument 100 eine Kopplungseinrichtung 142 zum wahlweisen direkten oder indirekten Koppeln der Kraftübertragungseinrichtung 120 mit mindestens einem der mindestens zwei Werkzeugelemente 134, 136 umfasst, kann die von dem Chirurg ausgeübte Betätigungskraft besonders direkt und ohne elastische Verformung der Führungseinrichtung 104 und/oder der Kraftübertragungseinrichtung 120 von dem proximalen Ende 124 der Führungseinrichtung 104 zu dem distalen Ende 130 der Führungseinrichtung 104 übertragen werden. Der Chirurg hat dadurch eine besonders vorteilhafte taktile Rückmeldung von den Werkzeugen 106, 108.

## Patentansprüche

1. Chirurgisches Instrument (100), umfassend mindestens zwei relativ zueinander bewegbare Werkzeugelemente (134, 136), welche an einem distalen Ende (130) einer Führungseinrichtung (104) des chirurgischen Instruments (100) angeordnet sind, und eine Kraftübertragungseinrichtung (120) zum Übertragen einer Betätigungskraft von einem proximalen Ende der Kraftübertragungseinrichtung (120) zu einem distalen Ende (148) der Kraftübertragungseinrichtung (120) zum Bewegen mindestens eines der mindestens zwei Werkzeugelemente (134, 136) relativ zu der Führungseinrichtung (104), mit einer Kopplungseinrichtung (142) zum wahlweisen direkten oder indirekten Koppeln der Kraftübertragungseinrichtung (120) mit mindestens einem der mindestens zwei Werkzeugelemente (134, 136), **dadurch gekennzeichnet, dass** die Kraftübertragungseinrichtung (120) aus einer ersten Kopplungsstellung, in welcher die Kraftübertragungseinrichtung (120) mit einem ersten Werkzeugelement (134, 136) des chirurgischen Instruments (100) gekoppelt ist, in eine zweite Kopplungsstellung, in welcher die Kraftübertragungseinrichtung (120) mit einem zweiten Werkzeugelement (134, 136) des chirurgischen Instruments (100) gekoppelt ist, bringbar ist.

2. Chirurgisches Instrument nach Anspruch 1, **dadurch gekennzeichnet, dass** die Kopplungseinrichtung (142) mindestens eine Führungsbahn (156, 160) zum Führen mindestens eines bewegbaren Werkzeugelements (134, 136) umfasst.

3. Chirurgisches Instrument nach einem der Ansprüche 1 oder 2, **dadurch gekennzeichnet, dass** das chirurgische Instrument (100) eine Betätigungseinrichtung (128) zum Wechseln einer Kopplungsstellung der Kopplungseinrichtung (142) umfasst.

4. Chirurgisches Instrument nach einem der Ansprüche 1 bis 3, **dadurch gekennzeichnet, dass** die Führungseinrichtung (104) zumindest teilweise elektrisch leitend ausgebildet ist.

5. Chirurgisches Instrument nach einem der Ansprüche 1 bis 4, **dadurch gekennzeichnet, dass** die Kraftübertragungseinrichtung (120) wahlweise ausschließlich mit einem ersten Werkzeugelement (134, 136) oder mit einem zweiten Werkzeugelement (134, 136) koppelbar ist.

6. Chirurgisches Instrument nach einem der Ansprüche 1 bis 5, **dadurch gekennzeichnet, dass** das chirurgische Instrument (100) eine elektrische Umschalteinrichtung (178) zum wahlweisen Beaufschlagen mindestens eines der mindestens zwei Werkzeugelemente (134, 136) mit elektrischer Energie umfasst.

7. Chirurgisches Instrument nach einem der Ansprüche 1 bis 6, **dadurch gekennzeichnet, dass** die Kopplungseinrichtung (142) zumindest abschnittsweise elektrisch leitend ausgebildet ist zum Übertragen eines elektrischen Stroms von der Kraftübertragungseinrichtung (120) auf mindestens eines der mindestens zwei Werkzeugelemente (134, 136).

8. Chirurgisches Instrument nach Anspruch 7, **dadurch gekennzeichnet, dass** die Kopplungseinrichtung (142) ausgebildet ist zum Übertragen eines elektrischen Stroms von der Kraftübertragungseinrichtung (120) auf das erste Werkzeugelement (134, 136) in einer ersten Kopplungsstellung der Kraftübertragungseinrichtung (120), in welcher die Kraftübertragungseinrichtung (120) mit einem ersten Werkzeugelement (134, 136) gekoppelt ist, und von der Kraftübertragungseinrichtung (120) auf das zweite Werkzeugelement (134, 136) in einer zweiten Kopplungsstellung, in welcher die Kraftübertragungseinrichtung (120) mit einem zweiten Werkzeugelement (134, 136) gekoppelt ist.

9. Chirurgisches Instrument nach einem der Ansprüche 1 bis 8, **dadurch gekennzeichnet, dass** die Kopplungseinrichtung (142) mindestens ein Kopplungsglied (152, 154) umfasst, welches mit mindestens einem Kopplungsglied (150) der Kraftübertragungseinrichtung (120) in Eingriff bringbar ist zum Herstellen einer formschlüssigen Verbindung zwischen der Kopplungseinrichtung (142) und der Kraftübertragungseinrichtung (120) in einer Kraftübertragungsrichtung (122) der Kraftübertragungseinrichtung (120).

10. Chirurgisches Instrument nach einem der Ansprüche 1 bis 9, **dadurch gekennzeichnet, dass** die Kopplungseinrichtung (142) mindestens eine Kopplungshilfe (153) zum Vereinfachen der Kopplung der Kraftübertragungseinrichtung (120) mit mindestens einem der mindestens zwei Werkzeugelemente (134, 136) aufweist.

11. Chirurgisches Instrument nach einem der Ansprüche 1 bis 10, **dadurch gekennzeichnet, dass** die Kopplungseinrichtung (142) mindestens ein Kopplungselement (144, 146) zum Koppeln der Kraftübertragungseinrichtung (120) mit mindestens einem der mindestens zwei Werkzeugelemente (134, 136) umfasst.

12. Chirurgisches Instrument nach Anspruch 11, **dadurch gekennzeichnet, dass** mindestens ein Kopplungselement (144, 146) in einer Kraftübertragungsrichtung (122) der Kraftübertragungseinrichtung (120) verschieblich an der Führungseinrichtung (104) gelagert ist.

13. Chirurgisches Instrument nach einem der Ansprüche 1 bis 12, **dadurch gekennzeichnet, dass** das chirurgische Instrument (100) mindestens ein bezüglich der Führungseinrichtung (104) feststehendes Werkzeugelement (132) umfasst.

14. Chirurgisches Instrument nach einem der Ansprüche 1 bis 13, **dadurch gekennzeichnet, dass** das chirurgische Instrument (100) mindestens ein Isolierelement (176) zum elektrischen Isolieren der Kraftübertragungseinrichtung (120), der Führungseinrichtung (104), mindestens eines Kopplungselements (144, 146) und/oder mindestens eines Werkzeugelements (134, 136) umfasst.

15. Chirurgisches Instrument nach einem der Ansprüche 1 bis 14, **gekennzeichnet durch** eine Verriegelungsvorrichtung zur Verriegelung des gerade nicht benutzten mindestens einen Werkzeugelements (134, 136).

## Claims

1. Surgical instrument (100), comprising at least two tool elements (134, 136) that are movable relative to one another, which are arranged at a distal end (130) of a guide device (104) of the surgical instrument (100), and a force transmission device (120) for the transferring of an actuating force from a proximal end of the force transmission device (120) to a distal end (148) of the force transmission device (120) for the purposes of moving at least one of the at least two tool elements (134, 136) relative to the guide device (104), with a coupling device (142) for selectively coupling the force transmission device (120) directly or indirectly to at least one of the at least two tool elements (134, 136), **characterized in that** the force transmission device (120) is moveable from a first coupling position in which the force transmission device (120) is coupled to a first tool element (134, 136) of the surgical instrument (100), into a second coupling position in which the force transmission device (120) is coupled to a second tool element (134, 136) of the surgical instrument (100).

2. Surgical instrument according to claim 1, **characterized in that** the coupling device (142) comprises at least one guideway (156, 160) for guiding at least one movable tool element (134, 136).

3. Surgical instrument according to any one of claims 1 or 2, **characterized in that** the surgical instrument (100) comprises an actuating device (128) for changing a coupling position of the coupling device (142).

4. Surgical instrument according to any one of claims 1 to 3, **characterized in that** the guide device (104) is at least partially electrically conductive.

5. Surgical instrument according to any one of claims 1 to 4, **characterized in that** the force transmission device (120) is arranged to be selectively coupled exclusively to a first tool element (134, 136) or to a second tool element (134, 136).

6. Surgical instrument according to any one of claims 1 to 5, **characterized in that** the surgical instrument (100) comprises an electrical switching device (178) for selectively applying electrical energy to at least one of the at least two tool elements (134, 136).

7. Surgical instrument according to any one of claims 1 to 6, **characterized in that** the coupling device (142) is electrically conductive at least in sections thereof for the purposes of the conveying of an electric current from the force transmission device (120) to at least one of the at least two tool elements (134, 136).

8. Surgical instrument according to claim 7, **characterized in that** the coupling device (142) is configured for the conveying of an electric current from the force transmission device (120) to the first tool element (134, 136) in a first coupling position of the force transmission device (120) in which the force transmission device (120) is coupled to a first tool element (134, 136), and from the force transmission device (120) to the second tool element (134, 136) in a second coupling position in which the force transmission device (120) is coupled to a second tool element (134, 136).

9. Surgical instrument according to any one of claims 1 to 8, **characterized in that** the coupling device (142) comprises at least one coupling member (152, 154) which is moveable into engagement with at least one coupling member (150) of the force transmission device (120) for producing a positive connection between the coupling device (142) and the force transmission device (120) in a force transmission direction (122) of the force transmission device (120).

10. Surgical instrument according to any one of claims 1 to 9, **characterized in that** the coupling device (142) has at least one coupling aid (153) for simplifying the coupling of the force transmission device (120) to at least one of the at least two tool elements (134, 136).

11. Surgical instrument according to any one of claims 1 to 10, **characterized in that** the coupling device (142) comprises at least one coupling element (144, 146) for coupling the force transmission device (120) to at least one of the at least two tool elements (134, 136).

12. Surgical instrument according to claim 11, **characterized in that** at least one coupling element (144, 146) is mounted on the guide device (104) such as to be displaceable in a force transmission direction (122) of the force transmission device (120).

13. Surgical instrument according to any one of claims 1 to 12, **characterized in that** the surgical instrument (100) comprises at least one tool element (132) which is fixed with respect to the guide device (104).

14. Surgical instrument according to any one of claims 1 to 13, **characterized in that** the surgical instrument (100) comprises at least one insulating element (176) for electrically insulating the force transmission device (120), the guide device (104), at least one coupling element (144, 146) and/or at least one tool element (134, 136).

15. Surgical instrument according to any one of claims 1 to 14, **characterized by** a latching device for latching the at least one tool element (134, 136) that is not currently being used.

## Revendications

1. Instrument chirurgical (100) comprenant au moins deux éléments d'outil (134, 136), qui peuvent être déplacés relativement l'un par rapport à l'autre et sont agencés à une extrémité distale (130) d'un dispositif de guidage (104) de l'instrument chirurgical (100), et comprenant un dispositif de transmission de force (120) pour transmettre une force d'actionnement à partir d'une extrémité proximale du dispositif de transmission de force (120) à une extrémité distale (148) du dispositif de transmission de force (120) en vue de déplacer au moins l'un desdits au moins deux éléments d'outil (134, 136) par rapport au dispositif de guidage (104), l'instrument comprenant en outre un dispositif de couplage (142) pour assurer sélectivement le couplage direct ou indirect du dispositif de transmission de force (120) à l'un au moins desdits au moins deux éléments d'outil (134, 136), **caractérisé en ce que** le dispositif de transmission de force (120) peut être amené, d'une première position de couplage, dans laquelle le dispositif de transmission de force (120) est couplé à un premier élément d'outil (134, 136) de l'instrument chirurgical (100), à une deuxième position de couplage, dans laquelle le dispositif de transmission de force (120) est couplé à un deuxième élément d'outil (134, 136) de l'instrument chirurgical (100).

2. Instrument chirurgical selon la revendication 1, **caractérisé en ce que** le dispositif de couplage (142) comprend au moins une voie de guidage (156, 160) pour guider au moins un élément d'outil (134, 136) mobile.

3. Instrument chirurgical selon l'une des revendications 1 ou 2, **caractérisé en ce que** l'instrument chirurgical (100) comprend un dispositif d'actionnement (128) pour changer une position de couplage du dispositif de couplage (142).

4. Instrument chirurgical selon l'une des revendications 1 à 3, **caractérisé en ce que** le dispositif de guidage (104) est d'une configuration au moins partiellement conductrice sur le plan électrique.

5. Instrument chirurgical selon l'une des revendications 1 à 4, **caractérisé en ce que** le dispositif de transmission de force (120) peut sélectivement être couplé exclusivement à un premier élément d'outil (134, 136) ou à un deuxième élément d'outil (134, 136).

6. Instrument chirurgical selon l'une des revendications 1 à 5, **caractérisé en ce que** l'instrument chirurgical (100) comprend un dispositif de commutation électrique (178) pour alimenter sélectivement au moins l'un desdits au moins deux éléments d'outil (134, 136) avec de l'énergie électrique.

7. Instrument chirurgical selon l'une des revendications 1 à 6, **caractérisé en ce que** le dispositif de couplage (142) est d'une configuration au moins partiellement conductrice sur le plan électrique, en vue d'assurer la transmission d'un courant électrique du dispositif de transmission de force (120) à au moins l'un desdits au moins deux éléments d'outil (134, 136).

8. Instrument chirurgical selon la revendication 7, **caractérisé en ce que** le dispositif de couplage (142) est conçu pour la transmission d'un courant électrique du dispositif de transmission de force (120) au premier élément d'outil (134, 136), dans une première position de couplage du dispositif de transmission de force (120), dans laquelle le dispositif de transmission de force (120) est couplé à un premier élément d'outil (134, 136), et du dispositif de transmission de force (120) au deuxième élément d'outil (134, 136), dans une deuxième position de couplage dans laquelle le dispositif de transmission de force (120) est couplé à un deuxième élément d'outil (134, 136).

9. Instrument chirurgical selon l'une des revendications 1 à 8, **caractérisé en ce que** le dispositif de couplage (142) comporte au moins un organe de couplage (152, 154), qui peut être amené en prise avec au moins un organe de couplage (150) du dispositif de transmission de force (120) pour établir une liaison par complémentarité de formes entre le dispositif de couplage (142) et le dispositif de transmission de force (120) dans une direction de transmission de force (122) du dispositif de transmission de force (120).

10. Instrument chirurgical selon l'une des revendications 1 à 9, **caractérisé en ce que** le dispositif de couplage (142) comprend au moins une aide au couplage (153) pour simplifier le couplage du dispositif de transmission de force (120) à l'un au moins desdits au moins deux éléments d'outil (134, 136).

11. Instrument chirurgical selon l'une des revendications 1 à 10, **caractérisé en ce que** le dispositif de couplage (142) comprend au moins un élément de couplage (144, 146) pour assurer le couplage du dispositif de transmission de force (120) à l'un au moins desdits au moins deux éléments d'outil (134, 136).

12. Instrument chirurgical selon la revendication 11, **caractérisé en ce qu'**au moins un élément de couplage (144, 146) est monté coulissant sur le dispositif de guidage (104), dans une direction de transmission de force (122) du dispositif de transmission de force (120).

13. Instrument chirurgical selon l'une des revendications 1 à 12, **caractérisé en ce que** l'instrument chirurgical (100) comprend au moins un élément d'outil (132) en position fixe par rapport au dispositif de guidage (104).

14. Instrument chirurgical selon l'une des revendications 1 à 13, **caractérisé en ce que** l'instrument chirurgical (100) comporte au moins un élément d'isolation (176) pour isoler électriquement le dispositif de transmission de force (120), le dispositif de guidage (104), au moins un élément de couplage (144, 146) et/ou au moins un élément d'outil (134, 136).

15. Instrument chirurgical selon l'une des revendications 1 à 14, **caractérisé par** un dispositif de verrouillage pour verrouiller ledit au moins un élément d'outil (134, 136) qui n'est pas utilisé à l'instant.
